# EUROPÄISCHE PATENTSCHRIFT

(11) **EP 1 021 502 B1**
(45) Veröffentlichungstag und Bekanntmachung des Hinweises auf die Patenterteilung: **12.11.2003**
(21) Anmeldenummer: 98958221.8
(22) Anmeldetag: 25.09.1998
(51) Int. Cl.: C11D 1/645, A61K 7/50, C11D 1/62

(54) **DETERGENSGEMISCHE**
DETERGENT MIXTURES
MELANGES DETERGENTS

(30) Priorität: 06.10.1997 DE 19743687
(43) Veröffentlichungstag der Anmeldung: 26.07.2000
(73) Patentinhaber: Cognis Deutschland GmbH & Co. KG, 40589 Düsseldorf (DE)
(72) Erfinder: BIGORRA, Joaquin, E-08203 Sabadell (ES); BONASTRE GILABERT, Nuria, E-08210 Barberá del Vallés (ES); PI SUBIRANA, Rafael, E-08400 Granollers (ES)
(86) Internationale Anmeldenummer: EP9806115
(87) Internationale Veröffentlichungsnummer: WO99018178

(56) Entgegenhaltungen:
- EP-A- 0 634 475
- EP-A- 0 786 250
- WO-A-98/41604
- DE-C- 19 539 846

## Beschreibung

### Gebiet der Erfindung

Die Erfindung betrifft Detergensgemische, enthaltend oligomere Esterquats und alkoxylierte Fettsäure-amidoamine sowie die Verwendung der Mischungen zur Herstellung von Wäscheweichspülmitteln und Haarpflegemitteln.

### Stand der Technik

Im Zuge gestiegener Anforderungen an die biologische Abbaubarkeit haben quartäre Fettsäurealkoanolimestersalze, die sogenannten "Esterquats", Tetraalkylammoniumverbindungen als kationische Tenside sowohl für die Herstellung Wäscheweichspülmitteln als auch für die Haaravivage verdrängt. Übersichten zu diesem Thema sind beispielsweise von R.Puchta et al. in **Tens.Surf.Det., 30, 186(1993)**, M.Brock in **Tens.Surf.Det. 30, 394 (1993),** R.Lagerman et al. in **J.Am.Oil.Chem.Soc., 71, 97 (1994)** sowie I.Sh**apiro in Cosm.Toil. 109, 77 (1994)** erschienen. Im Markt besteht jedoch der Wunsch nach Produkten mit weiter verbesserten Eigenschaften.

Die EP 0 786 250 A1 beschreibt a) avivierende Zubereitungen, die eine Wirkstoffkombination aus Esterquats und Alkylamidoaminen enthält. Die Schrift offenbart aber keine ethoxylierten Alkylamidoamine. Eine Mischung aus alkoxylierten Fettsäureamidoaminen und monomeren Esterquats ist in der **EP 0 634 475 A2** offenbart.

Die komplexe Aufgabe der Erfindung hat somit darin bestanden, Detergensgemische zur Verfügung zu stellen, die über verbesserte avivierende und antistatische Eigenschaften sowie vollständige bio-logische Abbaubarkeit verfügen, sich auch in kaltem Wasser rasch und vollständig dispergieren lassen, eine ausreichend hohe Viskosität besitzen, jedoch bei Lagerung nicht eindicken und Gele bilden, und schließlich ohne Mitverwendung von Alkoholen in beliebigem Verhältnis mit Wasser klar löslich sind.

### Beschreibung der Erfindung

Gegenstand der Erfindung sind Detergensgemische, enthaltend
(a) oligomere Esterquats, erhältlich durch Kondensation von Gemischen aus Mono- und Dicarbonsäuren mit Alkanolaminen und nachfolgender Quaternierung der Alkanolaminoligoester, und
(b) Anlagerungsprodukte von Alkylenoxiden an Fettsäureamidoamine,
mit der Maßgabe, dass die Komponenten (a) und (b) im Gewichtsverhältnis 10 : 90 bis 90 : 10 zugegeben sind.

Überraschenderweise wurde gefunden, dass die erfindungsgemäßen Gemische nicht nur hochkorizentrier wasserklar vorliegen, sondern auch eine vorteilhaft hohe und stabile Viskosität aufweisen. Die Zubereitungen lassen sich in beliebigen Mengen mit Wasser verdünnen und können dann sofort als Wäscheweichspül- oder Haarpflegemittel eingesetzt werden. Synthetischen wie natürlichen Fasern verleihen sie einen besonders angenehmen Weichgriff, zudem reduzieren sie die statische Aufladung zwischen den Faserfilamenten ganz erheblich. Ein weiterer Vorteil besteht darin, dass sie in kaltem Wasser besonders leicht dispergierbar und zudem leicht biologisch abbaubar sind.

### Oligomere Esterquats

Die oligomeren Esterquats besitzen mindestens zwei kationische Zentren und unterscheiden sich daher von anderen bekannten Esterquattypen, die nur über einen quartären Stickstoff verfugen. Die Herstellung und Verwendung dieser Stoffe ist aus der Deutschen Patentschrift **DE-C1 19539846** (Henkel) bekannt. Das Syntheseprinzip besteht vereinfacht dargestellt darin, mehrere, vorzugsweise aber genau zwei mehrwertige Alkanolamine mit Hilfe einer Dicarbonsäure zu verknüpfen, die freien Hydroxylgruppen mit Monocarbonsäuren ganz oder teilweise zu verestern und anschließend in an sich bekannter Weise die im oligomeren bzw. dimeren Ester vorhandenen Stickstoffatome zu quaternieren.

Typische Beispiele für Monocarbonsäuren, die als eine der beiden Säurekomponenten der oligomeren Esterquats in Frage kommen, sind Fett- bzw. Oxocarbonsäuren mit 6 bis 22 Kohlenstoffatomen wie beispielsweise Capronsäure, Caprylsäure, 2-Ethylhexansäure, Caprinsäure, Laurinsäure, Isotridecan-säure, Myristinsäure, Palmitinsäure, Palmoleinsäure, Stearinsäure, Isostearinsäure, Ölsäure, Elaidin-säure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure, vor-zugsweise in gehärteter bzw. teilgehärteter Form. Beispiele für geeignete Dicarbonsäuren, die als zweite, verbrückende Carbonsäurekomponente eingesetzt werden können, sind Bernsteinsäure, Maleinsäure, Glutarsäure, 1,12-Dodecandisäure und insbesondere Adipinsäure. Vorzugsweise werden oligomere Esterquats auf Basis von Fettsäuren mit 6 bis 22 Kohlenstoffatomen und Adipinsäure eingesetzt. Zur Herstellung der oligomeren Esterquats können die Mono- und Dicarbonsäuren im molaren Verhältnis 1:1 bis 3 : 1, vorzugsweise 1,5 : 1 bis 2,5 : 1 eingesetz werden.

Die Alkanolaminkomponente der oligomeren Esterquats kann sich von Methyldiethanolamin, vorzugsweise aber Triethanolamin sowie Gemischen der beiden ableiten. Zur Herstellung der Verbindungen kann man die Mono-/Dicarbonsäuren einerseits und die Alkanolamine andererseits im molaren Verhältnis 1 : 1 bis 3 : 1, vorzugsweise 1,5 : 1 bis 2 : 1 einsetzen,

Die Veresterung kann in an sich bekannter Weise durchgeführt werden, wie sie beispielsweise in der Internationalen Patentanmeldung **WO 91/01295** (Henkel) beschrieben wird. Vorteilhafterweise erfolgt die Veresterung bei Temperaturen von 120 bis 220 und insbesondere 130 bis 170°C und Drücken von 0,01 bis 1 bar. Als geeignete Katalysatoren haben sich hypophosphorige Säure bzw. deren Alkalisalze, vorzugsweise Natriumhypophosphit bewährt, die in Mengen von 0,01 bis 0,1 und vorzugsweise 0,05 bis 0,07 Gew.-% - bezogen auf die Einsatzstoffe - eingesetzt werden können. Im Hinblick auf eine besonders hohe Farbqualität und -stabilität hat sich die Mitverwendung von Alkali- und/ oder Erdalkaliborhydriden, wie beispielsweise Kalium-, Magnesium- und insbesondere Natriumborhydrid als vorteilhaft erwiesen. Die Co-Katalysatoren setzt man üblicherweise in Mengen von 50 bis 1000 und insbesondere 100 bis 500 ppm - wieder bezogen auf die Einsatzstoffe - ein. Entsprechende Verfahren sind auch Gegenstand der beiden Deutschen Patentschriften **DE-C1 4308792** und **DE-C1 4409322** (Henkel), auf deren Lehren hiermit ausdrücklich Bezug genommen wird. Es ist möglich, Mischungen der Fettsäuren und Dicarbonsäuren einzusetzen oder aber die Veresterung mit den beiden Komponenten nacheinander durchzuführen.

Die Quaternierung der Fettsäure/Dicarbonsäurealkanolaminester kann in an sich bekannter Weise durchgeführt werden. Obschon die Umsetzung mit den Alkylierungsmitteln auch in Abwesenheit von Lösungsmitteln durchgeführt werden kann, empfiehlt sich die Mitverwendung zumindest von geringen Mengen Wasser oder niederen Alkoholen, vorzugsweise Isopropylalkohol, zur Herstellung von Konzentraten, die einen Feststoffanteil von mindestens 80 und insbesondere mindestens 90 Gew.-% aufweisen. Als Alkylierungsmittel kommen Alkylhalogenide wie beispielsweise Methylchlorid, Dialkylsulfate wie beispielsweise Dimethylsulfat oder Diethylsulfat oder Dialkylcarbonate wie beispielsweise Dimethylcarbonat oder Diethylcarbonat in Frage. Üblicherweise werden die Ester und die Alkylietungsmittel im molaren Verhältnis 1 : 0,95 bis 1 : 1,05, also annähernd stöchiometrisch eingesetzt. Die Reaktionstemperatur liegt gewöhnlich bei 40 bis 80 und insbesondere bei 50 bis 60°C. Im Anschluß an die Reaktion empfiehlt es sich, nichtumgesetztes Alkylierungsmittel durch Zugabe beispielsweise von Ammoniak, einem (Alkanol)amin, einer Aminosäure oder einem Oligopeptid zu zerstören, wie dies beispielsweise in der Deutschen Patentanmeldung **DE-A14026184** (Henkel) beschrieben wird.

Bei der Umsetzung der Alkanolamine mit den Gemischen aus Mono- und Dicarbonsäuren fallen komplexe Gemische an, die ganz überwiegend Dimere enthalten, also Spezies, bei denen zwei Alkanolamine über eine Dicarbonsäure verbrückt werden und deren freie Hydroxylgruppen mit Monocarbonsäuren teilverestert vorliegen. Daneben werden nach Gelpermeationschromatographie auch oligomere Ester gefunden, in denen 3 bis 4 quartäre Zentren enthalten sind. Monomere Verbindungen mit nur einem quartären Stickstoff sind aus anwendungstechnischen Gründen unerwünscht, weil sie die Klarlöslichkeit beeinträchtigen. Ihr Auftreten läßt sich anwendungstechnisch nicht völlig vermeiden, bei Anwesenheit einer ausreichenden Menge Dicarbonsäure kann ihr Gehalt aber unter 5 Gew.-% gebracht werden. Die dimeren Verbindungen folgen der Formel (**I**), 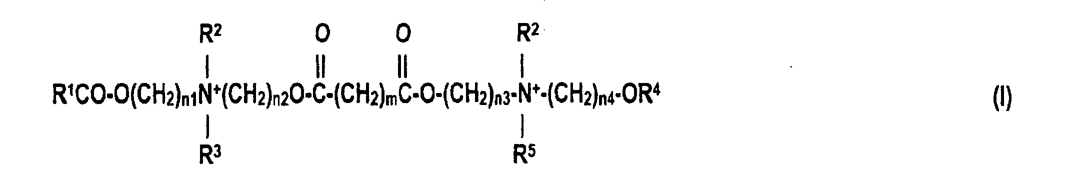 in der R¹CO für einen aliphatischen, linearen oder verzweigten, gesättigten oder ungesättigten Acylrest mit 6 bis 22 Kohlenstoffatomen, R² für einen Alkylrest mit 1 bis 4 Kohlenstoffatomen, R³ und R⁵ unabhängig voneinander für Alkyl- oder Hydroxyalkylreste mit 1 bis 4 Kohlenstoffatomen, R⁴ für Wasserstoff oder R¹CO, n1, n2, n3 und n4 unabhängig voneinander für Zahlen von 1 bis 5 und m für Zahlen von 1 bis 10 steht. Dimere Esterquats mit besonders vorteilhaften Eigenschaften folgen der Formel (1), in der R¹CO für einen linearen, gesättigten Acylrest mit 12 bis 18 Kohlenstoffatomen, R² für einen Methylrest, R³ und R⁵ jeweils für Hydroxyethylreste, R⁴ für R¹CO, n1, n2, n3 und n4 jeweils für 2 und m für 4 steht. Vorzugsweise enthalten die oligomeren Esterquats 20 bis 90, insbesondere 40 bis 80 Gew.-% Dimere, 10 bis 80 Gew.-%, insbesondere 20 bis 60 Gew.-% Oligomere und unter 5 Gew.-% Monomere, mit der Maßgabe, daß sich die Mengenangaben zu 100 Gew.-% addieren.

### Alkoxylierte Fettsäureamidoamine

Alkoxylierungsprodukte von Fettsäureamidoaminen sind ebenfalls literaturbekannt und folgen vorzugsweise der Formel (II), 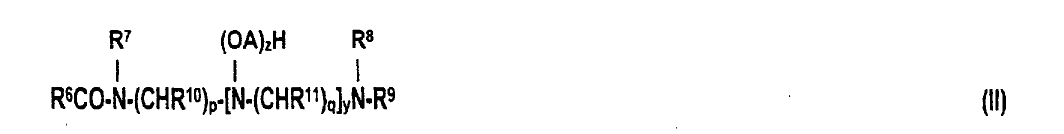 in R⁶CO für einen aliphatischen, linearen oder verzweigten, gesättigten oder ungesättigten Acylrest mit 6 bis 22 Kohlenstoffatomen, R⁷ und R⁸ unabhängig voneinander für Wasserstoff oder einen gegebenenfalls hydroxysubstituierten Alkylrest mit 1 bis 4 Kohlenstoffatomen, R⁹ für Wasserstoff, einen Alkylrest mit 1 bis 4 Kohlenstoffatomen oder R⁶CO, R¹⁰ und R¹¹ unabhängig voneinander für Wasserstoff oder eine Methylgruppe, A für eine lineare oder verzweigte Alkylengruppe mit 2 bis 4 Kohlenstoffatomen, p und q unabhängig voneinander für Zahlen von 1 bis 3, y für Zahlen von 1 bis 3 und z für Zahlen von 1 bis 20 steht.

Zu ihrer Herstellung geht man üblicherweise von Dialkylentriaminen oder Trialkylentetraminen aus, die zunächst mit 1 bis 2 Mol Carbonsäure verestert und dann in an sich bekannter Weise unter Insertion in die freien NH-Bindungen mit Alkylenoxiden, vorzugsweise Ethylenoxid umgesetzt werden. Typische Beispiele für geeignete Oligoamine sind Diethylentriamin, Dipropylentriamin, Triethylentetramin und Tripropylentetramin sowie deren Gemische. Als Carbonsäuren kommen die Fettsäuren mit 6 bis 22 Kohlenstoffatomen in Frage, wie z.B. Capronsäure, Caprylsäure, 2-Ethylhexansäure, Caprinsäure, Laurinsäure, isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkernoder Talgfettsäure, vorzugsweise in gehärteter bzw. teilgehärteter Form. Es hat sich als besonders vorteilhaft erwiesen, die Carbonsäuren in solchen Mengen einzusetzen, daß im Durchschnitt Diamide resultieren. Aus anwendungstechnischen Gründen besonders bevorzugt sind des weiteren alkoxylierte Fettsäureamidoamine der Formel (**II**), in der R⁶CO für einen linearen, gesättigten Acylrest mit 12 bis 18 Kohlenstoffatomen, R⁷, R⁸, R¹⁰ und R¹¹ jeweils für Wasserstoff, R⁹ für R⁶CO, A für eine Ethylengruppe, p und q jeweils für 2, y für 1 und z für Zahlen von 5 bis 10 steht.

Die Alkoxylierung kann in an sich bekannter Weise erfolgen, d.h. Ethylenoxid, Propylenoxid oder deren Gemische werden in Gegenwart saurer, vorzugsweise aber basischer Katalysatoren, wie z.B. Natriummethylat oder calcinierter Hydrotalcit angelagert. Es entstehen nichtionische Verbindungen, die jedoch in saurer Lösung rasch protoniert werden und dann ein pseudokationisches Verhalten zeigen.

Im Sinne der Erfindung können die Detergensgemische die Komponenten (a) und (b) im Gewichtsverhältnis 10 : 90 bis 90 : 10, vorzugsweise 25 : 75 bis 75 : 25 und insbesondere 40 : 60 bis 60 : 40 enthalten.

### Gewerbliche Anwendbarkeit

Weitere Gegenstände der Erfindung bestehen in der Verwendung der Detergensgemische zur Herstellung von Wäscheweichspülmitteln und Zubereitungen für die Haarpflege, in denen sie in Mengen von 1 bis 50, vorzugsweise 5 bis 35 und insbesondere 10 bis 25 Gew.-% enthalten sein können. Abgesehen von der Möglichkeit, die Mischungen unmittelbar für den angegebenen Zweck zu verwenden, besteht die einfachste Anwendungsform darin, sie mit Wasser auf die gewünschte Anwendungskonzentration zu verdünnen.

### Tenside

In diesem Zusammenhang ist es möglich, den Zubereitungen auch weitere Zusatzstoffe hinzuzufügen, insbesondere weitere Tenside, die mit den Komponenten (a) und (b) kompatibel sind. Hierbei handelt es sich in erster Linie um weitere nichtionische oder kationische bzw. amphotere oder zwitterionische Tenside. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und monomere Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), " Surfactants in Consumer Products", Springer Verlag, Berlin, 1987, S. 54-124** oder **J.Falbe (ed.), "Katalysatoren, Tenside und Mineralöladditive ", Thieme Verlag, Stuttgart, 1978, S. 123-217** verwiesen.

### Hilfs- und Zusatzstoffe

In Abhängigkeit des Einsatzzweckes, also Textil- oder Haaravivage, können die unter Verwendung der Detergensgemische erhältlichen Zubereitungen weitere typische Hilfs- und Zusatzstoffe enthalten, als da sind: Ölkörper, Emulgatoren, Überfettungsmittel, Perlglanzwachse, Stabilisatoren, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Parfümöle, Farbstoffe und dergleichen:

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl* Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C₈/₁₈-Alkyl- oder -Acylgruppe im Molekül mindestens eine freie Aminogruppe und mindestens eine -COOH- oder -SO₃H- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau). quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylatkohol oder Partialglyceriden in Frage. Als **Stabilisatoren** können Metallsalze von Fettsäuren, wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als Quellmittef für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicyisäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin ;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigaikylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als Selbstbräuner eignet sich Dihydroxyaceton.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81.106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Herstellbeispiel.** In einem 2-I-Dreihalskolben mit Rührer, Tropftrichter und Destillationsaufsatz wurden 540 g (2 Mol) teilgehärtete Talgfettsäure, 292 g (2 Mol) Adipinsäure und 2 g Natriumhypophosphit bei 80°C vermischt. Es wurde ein Vakuum von 20 mm Hg angelegt und portionsweise 596 g (4 Mol) Triethanolamin zugegeben. Die Mischung wurde auf 170°C erhitzt, der Druck auf 5 mm Hg abgesenkt und solange Wasser aus dem Gleichgewicht entfernt, bis die Säurezahl auf einen Wert unter 5 abgesunken war. 500 g des resultierenden Esters (entsprechend 1,5 Äquivalenten) wurden in einen zweiten Kolben überführt und bei 50°C in 171 g Isopropylalkohol gelöst. Zu der Mischung wurden langsam 180 g (1,43 Mol) Dimethylsulfat gegeben; anschließend wurde bei 65°C über 5 h gerührt. Es resultierte eine viskose, klare Flüssigkeit mit einem Kationtensidgehalt von 1,2 meq/g und einem Trockenrückstand von 80,5 Gew.-%. Eine 5 Gew.-%ige Lösung in Wasser war klar und zeigte eine Gardnerfarbzahl von 1. Das Produkt ist unter der Bezeichnung Dehyquart D 6003 im Handel erhältlich,

**Anwendungstechnische Beispiele.** Es wurden sieben Detergensmischungen hergestellt und hinsichtlich Viskosität, Lagerstabilität, Löslichkeit, Dispergierbarkeit, Weichgriff und Naßkämmbarkeit untersucht. Die Viskosität wurde nach Brookfield in einem RVF-Viskosimeter (Spindel 1, 10 Upm) unmittelbar nach der Herstellung und nach 4-wöchiger Lagerung bei 40°C untersucht. Die Löslichkeit wurde nach der Herstellung, die Dispergierbarkeit (also die Stabilität der wäßrigen Zubereitungen) nach 1 h visuell beurteilt. Die Bestimmung des Weichgriffs erfolgte nach Zwangsapplikation der Testgemische auf Baumwollgewebe durch ein Panel von 6 erfahrenen Testern. Hierbei bedeutet (1) sehr weich, (2) weich, (3) hart und (4) sehr hart. Angegeben sind die Mittelwerte des Panels aus drei Testzyklen. Zur Bestimmung der Naßkämmbarkeit wurden die statische Aufladung zwischen den Faserfilamenten vor und nach Behandlung mit den Testlösungen bestimmt, die ein Maß für die Kämmarbeit darstellt. Die Mischungen 1 bis 3 sind erfindungsgemäß, die Mischungen V1 bis V4 dienen zum Vergleich. Die Ergebnisse sind in Tabelle 1 zusammengefaßt.

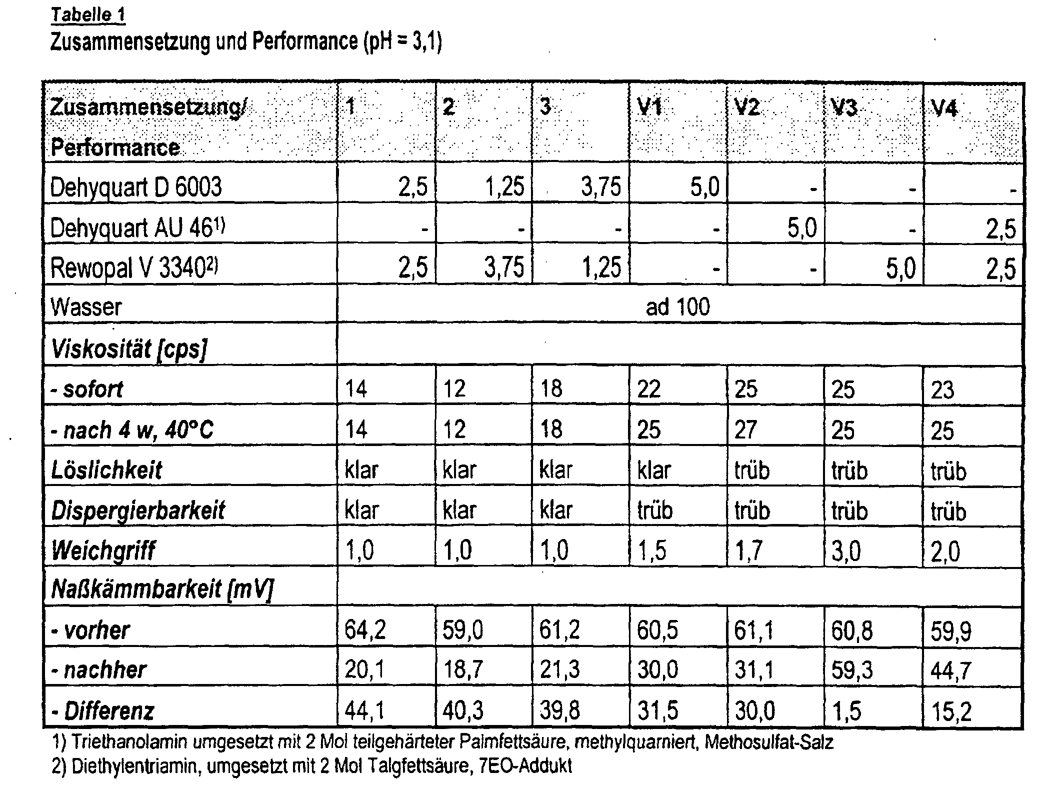

## Patentansprüche

1. Detergensgemische, enthaltend
(a) oligomere Esterquats, erhältlich durch Kondensation von Gemischen aus Mono- und Dicarbonsäuren mit Alkanolaminen und nachfolgende Quaternierung der Alkanolaminoligoester, und
(b) Anlagerungsprodukte von Alkylenoxiden an Fettsäureamidoamine,
mit der Maßgabe, daß die Komponenten (a) und (b) im Gewichtsverhältnis 10 : 90 bis 90 : 10 zugegen sind.

2. Detergensgemische nach Anspruch 1, **dadurch gekennzeichnet, daß** sie als Komponente (a) oligomere Esterquats auf Basis von Fettsäuren mit 6 bis 22 Kohlenstoffatomen und Adipinsäure enthalten.

3. Detergensgemische nach den Ansprüchen 1 und 2, **dadurch gekennzeichnet, daß** sie als Komponente (a) oligomere Esterquats auf Basis von Mono- und Dicarbonsäuren im molaren Verhältnis 1 : 1 bis 3 : 1 enthalten.

4. Detergensgemische nach den Ansprüchen 1 bis 3, **dadurch gekennzeichnet, daß** sie als Komponente (a) oligomere Esterquats auf Basis Triethanolamin enthalten.

5. Detergensgemische nach den Ansprüchen 1 bis 4, **dadurch gekennzeichnet, daß** sie als Komponente (a) oligomere Esterquats auf Basis von Mono-/Dicarbonsäuren und Alkanolaminen im molaren Verhältnis 1 : 1 bis 3 : 1 enthalten.

6. Detergensgemische nach den Ansprüchen 1 bis 5, **dadurch gekennzeichnet, daß** sie als Komponente (a) dimere Esterquats enthalten, die ganz oder überwiegend Formel (**I**) folgen, 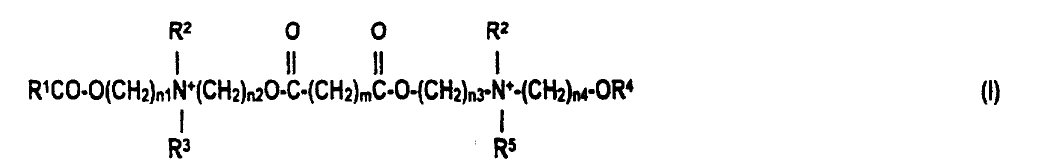 in der R¹CO für einen aliphatischen, linearen oder verzweigten, gesättigten oder ungesättigten Acylrest mit 6 bis 22 Kohlenstoffatomen, R² für einen Alkylrest mit 1 bis 4 Kohlenstoffatomen, R³ und R⁵ unabhängig voneinander für Alkyl- oder Hydroxyalkylreste mit 1 bis 4 Kohlenstoffatomen, R⁴ für Wasserstoff oder R¹CO, n1, n2, n3 und n4 unabhängig voneinander für Zahlen von 1 bis 5 und m für Zahlen von 1 bis 10 steht.

7. Detergensgemische nach Anspruch 6, **dadurch gekennzeichnet, daß** sie als Komponente (a) dimere Esterquats der Formel (**I**) enthalten, in der R¹CO für einen linearen, gesättigten Acylrest mit 12 bis 18 Kohlenstoffatomen, R² für einen Methylrest, R³ und R⁵ jeweils für Hydroxyethylreste, R⁴ für R¹CO, n1, n2, n3 und n4 jeweils für 2 und m für 4 steht.

8. Detergensgemische nach den Ansprüchen 1 bis 7, **dadurch gekennzeichnet, daß** sie als Komponente (b) alkoxylierte Fettsäureamidoamine der Formel (**II**) enthalten, 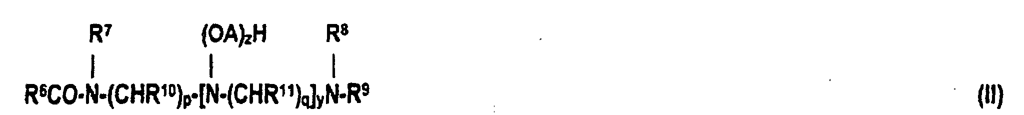 in R⁶CO für einen aliphatischen, linearen oder verzweigten, gesättigten oder ungesättigten Acylrest mit 6 bis 22 Kohlenstoffatomen, R⁷ und R⁸ unabhängig voneinander für Wasserstoff oder einen gegebenenfalls hydroxysubstituierten Alkylrest mit 1 bis 4 Kohlenstoffatomen, R⁹ für Wasserstoff, einen Alkylrest mit 1 bis 4 Kohlenstoffatomen oder R⁶CO, R¹⁰ und R¹¹ unabhängig voneinander für Wasserstoff oder eine Methylgruppe, A für eine lineare oder verzweigte Alkylengruppe mit 2 bis 4 Kohlenstoffatomen, p und q unabhängig voneinander für Zahlen von 1 bis 3, y für Zahlen von 1 bis 3 und z für Zahlen von 1 bis 20 steht.

9. Detergensgemische nach Anspruch 8, **dadurch gekennzeichnet, daß** sie als Komponente (b) Fettsäureamidoamine der Formel (**II**) enthalten, in der R⁶CO für einen linearen, gesättigten Acylrest mit 12 bis 18 Kohlenstoffatomen. R⁷, R⁸, R¹⁰ und R¹¹ jeweils für Wasserstoff, R⁹ für R⁶CO, A für eine Ethylengruppe, p und q jeweils für 2, y für 1 und z für Zahlen von 5 bis 10 steht.

10. Verwendung der Detergensgemische nach den Ansprüchen 1 bis 9 zur Herstellung von Wäscheweichspülmitteln.

11. Verwendung der Detergensgemische nach den Ansprüchen 1 bis 9 zu Herstellung von Haarpflegemitteln.

## Claims

1. Detergent mixtures containing
(a) oligomeric esterquats obtainable by condensation of mixtures of mono- and dicarboxylic acids with alkanolamines and subsequent quaternization of the alkanolamine oligoesters and
(b) products of the addition of alkylene oxides onto fatty acid amido-amines,
with the proviso that components (a) and (b) are present in a ratio by weight of 10:90 to 90:10

2. Detergent mixtures as claimed in claim 1, **characterized in that** they contain oligomeric esterquats based on fatty acids containing 6 to 22 carbon atoms and adipic acid as component (a).

3. Detergent mixtures as claimed in claims 1 and 2, **characterized in that** they contain oligomeric esterquats based on mono- and dicarboxylic acids in a molar ratio of 1:1 to 3:1 as component (a).

4. Detergent mixtures as claimed in claims 1 to 3, **characterized in that** they contain oligomeric esterquats based on triethanolamine as component (a).

5. Detergent mixtures as claimed in claims 1 to 4, **characterized in that** they contain oligomeric esterquats based on mono-/dicarboxylic adds and alkanolamines in a molar ratio of 1:1 to 3:1 as component (a).

6. Detergent mixtures as claimed in claims 1 to 5, **characterized in that** they contain as component (a) dimeric esterquats completely or predominantly corresponding to formula (**I**) : 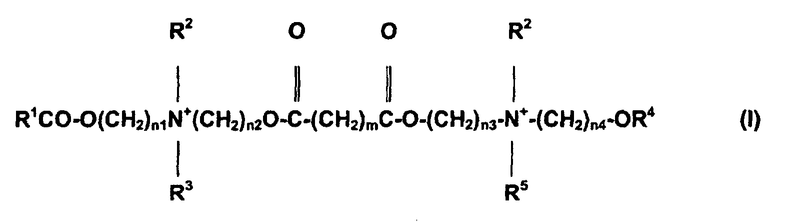 in which R¹CO is an aliphatic, linear or branched, saturated or unsaturated acyl group containing 6 to 22 carbon atoms, R² is an alkyl group containing 1 to 4 carbon atoms, R³ and R⁵ independently of one another represent alkyl or hydroxyalkyl groups containing 1 to 4 carbon atoms, R⁴ is hydrogen or has the same meaning as R¹CO, n1, n2, n3 and n4 independently of one another have a value of 1 to 5 and m has a value of 1 to 10.

7. Detergent mixtures as claimed in claim 6, **characterized in that** they contain as component (a) dimeric esterquats corresponding to formula (I) where R¹CO is a linear saturated acyl group containing 12 to 18 carbon atoms, R² is a methyl group, R³ and R⁵ are each hydroxyethyl groups, R⁴ has the same meaning as R¹CO, n1, n2, n3 and n4 each have a value of 2 and m has a value of 4.

8. Detergent mixtures as claimed in claims 1 to 7, **characterized in that** they contain as component (b) alkoxylated fatty acid amidoamines corresponding to formula (**II**): 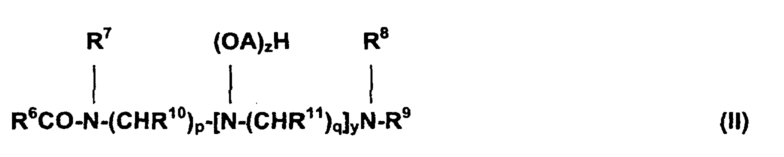 In which R⁶CO is an aliphatic, linear or branched, saturated or unsaturated acyl group containing 6 to 22 carbon atoms, R⁷ and R⁸ independently of one another represent hydrogen or an optionally hydroxysubstituted alkyl group containing 1 to 4 carbon atoms, R⁹ is hydrogen, an alkyl group containing 1 to 4 carbon atoms or has the same meaning as R⁶CO, R¹⁰ and R¹¹ independently of one another represent hydrogen or a methyl group, A is a linear or branched alkylene group containing 2 to 4 carbon atoms, p and q independently of one another have a value of 1 to 3, y has a value of 1 to 3 and z has a value of 1 to 20.

9. Detergent mixtures as claimed in claim 8, **characterized in that** they contain as component (b) fatty acid amidoamines corresponding to formula (11) where R⁶CO is a linear saturated acyl group containing 12 to 18 carbon atoms, R⁷, R⁸, R¹⁰ and R¹¹ each represent hydrogen, R⁹ has the same meaning as R⁶CO, A is an ethylene group, p and q each have a value of 2, y has the value 1 and z has a value of 5 to 10.

10. The use of the detergent mixtures claimed in claims 1 to 9 for the production of fabric softeners.

11. The use of the detergent mixtures claimed in claims 1 to 9 for the production of hair-care compositions.

## Revendications

1. Mélanges de détergents contenant
(a) des esterquats oligomères accessibles par condensation de mélanges à base d'acide mono et dicarboxyliques avec des alkanolamines et quaternisation subséquente de l'oligoester d'alkanolamine et
(b) des produits d'addition d'oxydes d'alkylène sur des amidoamines d'acide gras,
avec la précision que les composants (a) et (b) sont présents dans un rapport en poids de 10 : 90 à 90 : 10.

2. Mélanges de détergents selon la revendication 1,
**caractérisés en ce qu'**
ils renferment comme composant (a) des esterquats oligomères à base d'acides gras ayant de 6 à 22 atomes de carbone et d'acide adipique.

3. Mélanges de détergents selon la revendication 1 ou 2,
**caractérisés en ce qu'**
ils renferment comme composant (a) des esterquats oligomères à base d'acides mono- et dicarboxyliques dans un rapport molaire allant de 1 : 1 à 3 : 1.

4. Mélanges de détergents selon les revendications 1 à 3,
**caractérisés en ce qu'**
ils renferment comme composant (a) des esterquats oligomères à base de triéthanolamine.

5. Mélanges de détergents selon les revendications 1 à 4,
**caractérisés en ce qu'**
ils renferment comme composant (a) des esterquats oligomères à base d'acide mono- et dicarboxylique et d'alkanolamines dans un rapport polaire allant de 1 :1 à 3 :1.

6. Mélanges de détergents selon les revendications 1 à 5,
**caractérisés en ce qu'**
ils renferment comme composant (a) des esterquats dimères qui répondent totalement ou principalement à la forme (I) : 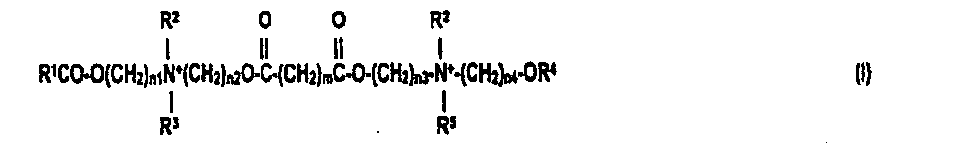 dans laquelle R¹CO représente un reste acyle aliphatique, linéaire ou ramifié, saturé ou non saturé, ayant de 6 à 22 atomes de carbone, R² représente un reste alkyle ayant de 1 à 4 atomes de carbone, R³ et R⁵ indépendamment l'un de l'autre représentent des restes alkyle ou hydroxyalkyle ayant de 1 à 4 atomes de carbone, R⁴ représente de l'hydrogène ou R¹CO, n1, n2, n3 et n4, indépendamment les uns des autres, représentent des nombres allant de 1 à 5 et m représente des nombres allant de 1 à 10.

7. Mélanges de détergents selon la revendication 6,
**caractérisés en ce qu'**
ils renferment comme composant (a) des esterquats dimères de formule (I) dans laquelle R¹CO représente un reste acyle linéaire, saturé ayant de 12 à 18 atomes de carbone, R² représente un reste méthyle, R³ et R⁵ respectivement représentent des restes hydroxyéthyle, R⁴CO représente R¹CO, n1, n2, n3 et n4 représentent respectivement 2 et m représente 4.

8. Mélanges de détergents selon les revendications 1 à 7,
**caractérisés en ce qu'**
ils renferment comme composant (b) des amidoamines d'acide gras alkoxylés de formule (II) 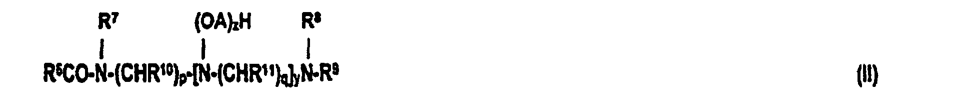 dans laquelle R⁶CO représente un reste acyle aliphatique, linéaire ou ramifié, saturé ou non saturé, ayant de 6 à 22 atomes de carbone, R⁷ et R⁸, indépendamment l'un de l'autre, représentent de l'hydrogène ou un reste alkyle éventuellement substitué par un hydroxy, ayant de 1 à 4 atomes de carbone, R⁹ représente de l'hydrogène, un reste alkyle ayant de 1 à 4 atomes de carbone ou R⁶CO, R¹⁰ et R¹¹ indépendamment l'un de l'autre, représentent de l'hydrogène ou un groupe méthyle, A représente un groupe alkylène linéaire ou ramifié ayant de 2 à 4 atomes de carbone, p et q indépendamment l'un de l'autre représentent des nombres allant de 1 à 3, y représente des nombres allant de 1 à 3 et z représente des nombres allant de 1 à 20.

9. Mélanges de détergents selon la revendication 8,
**caractérisés en ce qu'**
ils renferment comme composant (a) des amidoamines d'acide gras de formule (II) dans laquelle R⁶CO représente un reste acyle linéaire, saturé ayant de 12 à 18 atomes de carbone, R⁷, R⁸, R¹⁰ et R¹¹ respectivement représentent de l'hydrogène, R⁹ représente R⁶CO, A représente un groupe éthylène, p et q respectivement représentent 2, y représente 1 et z représente des nombres allant de 5 à 10.

10. Utilisation des mélanges de détergents selon les revendications 1 à 9, en vue de la préparation de produits de rinçage assouplissants pour les textiles.

11. Utilisation des mélanges de détergents selon les revendications 1 à 9, en vue de la préparation de produits de soins capillaires.
